# EUROPÄISCHE PATENTANMELDUNG

(11) **EP 3 903 864 A1**
(43) Veröffentlichungstag der Anmeldung: **03.11.2021**
(21) Anmeldenummer: 20171878.0
(22) Anmeldetag: 28.04.2020
(51) Int. Cl.: A61M 16/04, A61M 25/00, A61M 1/00

(54) **INTUBATIONSSCHLAUCH UND BEHANDLUNGSVORRICHTUNG**

(71) Anmelder: Carag AG, 6340 Baar (CH)
(72) Erfinder: LARSSON, Michael, 6302 Zug (CH); BERNHARD, Jérôme, 8003 Zürich (CH); CHRISTEN, Lukas, 6004 Luzern (CH)
(74) Vertreter: Grünecker Patent- und Rechtsanwälte PartG mbB

(57) **Zusammenfassung**

Die vorliegende Erfindung betrifft einen Intubationsschlauch (2) mit einem ersten Lumen (8) für einen Gasaustausch und einem zweiten Lumen (14) für die Steuerungen eines Cuffs (12), der von einem distalen Ende (10) des ersten Lumens (8) überragt ist, wobei der Intubationsschlauch (2) zur besseren Behandlung von Lungenkrankheiten zumindest einen Drainageschlauch (16) aufweist, der das distale Ende (10) des ersten Lumens (8) überragt und mit einem dritten Lumen (16) kommuniziert, das parallel zu dem ersten Lumen (8) verlegt ist. Die an den Intubationsschlauch (2) anschließbare erfindungsgemäße Vorrichtung zur Behandlung von Lungenkrankheiten hat ein Beatmungsgerät, das mit dem ersten Lumen (8) des Intubationsschlauches (2) verbindbar ist, eine Saugpumpe (32), die an zumindest einer Ventileinrichtung (24) angeschlossen ist, die die Saugpumpe (32) mit einer Schnittstelle (20) für den wenigstens einen Drainageschlauch (16) verbindet, und eine Steuerung (46) aufweist, die steuerungsmäßig mit der wenigstens einen Ventileinrichtung (24), der Saugpumpe (32) verbunden ist. Ferner betrifft die Erfindung eine Trachealkanüle (61) zur Behandlung von Lungenkrankheiten.

## Beschreibung

Die vorliegende Erfindung betrifft die Behandlung von Lungenkrankheiten und schlägt hierzu einen verbesserten Intubationsschlauch, eine dazu angepasst ausgebildete Behandlungsvorrichtung und eine Trachealkanüle zur Behandlung von Lungenkrankheiten.

Lungenerkrankungen, beispielsweise infektiöser Art, bedürfen mitunter der Beatmung des Patienten. Hierzu wird der Patient intubiert und über einen konventionellen Intubationsschlauch beatmet. Lungenerkrankungen können aber auch zu einer Überproduktion von Sekreten in der Lunge führen, die die zentrale Funktion der Lunge behindern, so dass Gasaustausch trotz künstlicher Beatmung nur unzureichend möglich ist. Beispiele für Erkrankungen solcher Art können Lungenödeme, COPD, cystische Fibrose oder andere schleimbildende oder flüssigkeitsbildende Lungenkrankheiten sein.

Hier will die vorliegende Erfindung Abhilfe schaffen und schlägt einen für die Behandlung von Lungenerkrankungen verbesserten angepassten Intubationsschlauch sowie eine Vorrichtung zur Behandlung von Lungenerkrankungen vor. Ferner schlägt die Erfindung eine Trachealkanüle zur Behandlung von Lungenkrankheiten.

Der erfindungsgemäße Intubationsschlauch hat ein erstes Lumen für einen Gasaustausch und ein zweites Lumen für die Steuerung eines Cuffs, der von einem distalen Ende des ersten Lumens überragt ist und zur Abdichtung des Intubationsschlauchs in der Trachea angepasst ausgebildet ist. Insoweit unterscheidet sich der erfindungsgemäße Intubationsschlauch nicht von einem üblichen Intubationsschlauch für die künstliche Beatmung.

Der erfindungsgemäße Intubationsschlauch hat indes zumindest einen, üblicherweise exakt zwei Drainageschläuche, der ein distales Ende des ersten Lumens überragt und mit einem parallel zu dem ersten Lumen verlegten zugeordneten dritten Lumen kommuniziert. Die Drainageschläuche haben bevorzugt einen Außendurchmesser von 2 mm bis 10 mm, insbesondere von 2 mm bis 5 mm. Denkbar ist ferner ein weiteres viertes Lumen für einen optischen Leiter, beispielsweise eine Kamera.

Im Rahmen der Beschreibung der vorliegenden Erfindung bezeichnet "distal" den bei der Benutzung des Intubationsschlauchs im Körper liegenden Endbereich des Intubationsschlauches, wohingegen "proximal" das aus dem Körper herausstehende Ende des Intubationsschlauches bezeichnet.

Das erste Lumen liegt auch bei dem erfindungsgemäßen Intubationsschlauch wie an sich bekannt in der Trachea, üblicherweise im unteren Ende der Trachea. Der bzw. die Drainageschläuche überragen dieses erste Lumen und sind hinsichtlich ihrer Länge und ihres Durchmessers so angepasst ausgebildet, dass jeder der Drainageschläuche in einen der Lungenflügel (bronchus principalis dexter bzw. sinister) eingebracht werden kann. Der Drainageschlauch dient dem Spülen der Lunge, wobei Schleim mit Salzlösungen, beispielsweise mit Enzymen versetzt, verflüssigt wird. Denkbar ist ebenfalls der Einsatz zur Therapie mittels Medikamente. Die Drainageschläuche sollen möglichst tief bis in die Lunge hineinragen. Der bzw. die Drainageschläuche sind über die ihnen zugeordneten dritten Lumen mit einer Saugpumpe und einer optionalen Spülpumpe verbunden. Eine Spülpumpe ist wahlweise nutzbar, da es denkbar erscheint, den Spülvorgang ohne Spülpumpe lediglich mit Hilfe der Gravitation durchzuführen. Jeder der Drainageschläuche kann über das ihm zugeordnete dritte Lumen wahlweise benutzt werden, um den linken oder den rechten Lungenflügel zu spülen. Das dritte Lumen kann dabei durch einen Schlauchkörper gebildet sein, der das erste und das zweite Lumen ausbildet und von dem der Drainageschlauch distal abgeht oder durch einen den Drainageschlauch ausbildenden Körper, der in dem Schlauchkörper verlegt ist. Dabei lässt sich die Erfindung von der Vorstellung leiten, dass üblicherweise lediglich ein Lungenflügel gespült wird, wohingegen der andere Lungenflügel zu dieser Zeit nicht gespült wird, um den Gasaustausch aufrecht zu erhalten. Die Drainageschläuche können jeweils mit einem Cuff versehen sein, der den Zugang zu dem entsprechenden Lungenflügel abdichtet, so dass hinter dem Cuff der Lungenflügel gespült werden kann, ohne dass dieses Spülen den anderen Lungenflügel beeinträchtigt. Ein Cuff kann im Sinne der Erfindung aufgeblasen werden. Er dichtet die Luftröhre ab und vermindert auch beispielsweise das Risiko einer Aspiration.

In einer Ausführungsform ist der zumindest eine Drainageschlauch in Verlaufsrichtung des dritten Lumens axial verschiebbar und vorzugsweise zusätzlich rotierbar. Dies ist von Vorteil, damit der Drainageschlauch möglichst kontrolliert im betroffenen Bereich der Lunge platziert werden kann. Das dritte Lumen wird dabei durch einen separaten Drainageschlauch körpergebildet, der innerhalb eines Grundkörpers verschieblich und drehbar geführt ist, der das erste und das zweite Lumen ausbildet. Dieser Grundkörper verwirklicht die für die Beatmung notwendigen Elemente.

Beim Spülen wird zunächst Spülflüssigkeit über die Drainageleitung eingeleitet. Danach wird die Spülflüssigkeit durch Absaugen durch die identische Drainageleitung wieder aus dem Lungenflügel entfernt. Das Spülen stellt dabei keinen Konflikt zu der Beatmung dar. Die Beatmung erfolgt mit einem Gasvolumenstrom von beispielsweise +/- 60 Liter pro Minute bei einem Druck von etwa 40 mbar. Es ist dabei denkbar, dass für das Spülen beispielsweise ein Volumenstrom von etwa 1 Liter pro Minute ausreichend ist. Das Spülen erfolgt dabei einerseits wechselweise zwischen den beiden Lungenflügeln und in der Regel intermittierend. Nach einem Spül- und Absaugzyklus wird der Lungenflügel über einen gewissen Zeitraum nicht mehr gespült.

Im Rahmen der Behandlung wird die Beatmung üblicherweise wie allgemein bekannt kontinuierlich durchgeführt. Dieser Beatmung überlagert werden Spülzyklen, bei denen über jeweils einen der Drainageschläuche Spülflüssigkeit in einen Lungenflügel eingebracht und gegebenenfalls nach einer gewissen Einwirkzeit wieder abgesogen wird. Als Spülflüssigkeit kann beispielsweise Kochsalzlösung zum Einsatz kommen, die in der Lunge befindlichen Schleim löst, so dass dieser beim Absaugen der Spülflüssigkeit zusammen mit der Spülflüssigkeit aus der Lunge entfemt werden kann. Die Spülflüssigkeit kann zusätzlich Enzyme, Antibiotika, Virostatika, Antiseptika oder andere geeignete entzündungshemmende Medikamente enthalten.

Hierdurch wird eine verbesserte Beatmung bewirkt. Der Gasaustausch in der Lunge ist zu einem reduzierten Anteil von Sekret in der Lunge behindert, sodass die Effektivität der Beatmung gesteigert wird.

Das Beatmungskonzept und auch der Intubationsschlauch nach der vorliegenden Erfindung unterscheiden sich von der aus WO 2017/086939 A1 bekannten Lösung, bei welcher ein Intubationsschlauch mehrere Lumen aufweist, die unter anderem auch dazu dienen, am Ende des Intubationsschlauches einen antiseptischen Nebel in die Lunge einzubringen und dabei zeitgleich den Nebel und von dem Nebel gelöstes Sekret aus der Lunge abzusaugen. Die aus diesem Stand der Technik bekannte Vorrichtung hat einen Zerstäuber, der mit hohem Druck Nebel aus einer antiseptischen Flüssigkeit erzeugt.

Während bei dem vorbekannten Stand der Technik das Einbringen von Flüssigkeit in die Lunge unbedingt vermieden werden soll und selbst der Nebel nur bei zeitgleichem Absaugen in die Lunge eingebracht wird, schlägt die vorliegende Erfindung Spülzyklen vor, bei denen zumindest ein Lungenflügel möglichst vollständig gespült wird, wozu die Spülflüssigkeit zunächst in den jeweiligen Lungenflügel eingeleitet und erst nach einer gewissen Einwirkzeit wieder abgesaugt wird.

Bei dem erfindungsgemäßen Intubationsschlauch ist das erste Lumen üblicherweise mit einer derartigen Länge ausgebildet, dass das erste Lumen im Bereich des unteren Endes der Trachea endet. In an sich bekannter Weise ist das erste Lumen dementsprechend in der Trachea, speziell in dem unteren Ende der Trachea verlegt und dort in der Regel über den Cuff abgedichtet. Die Drainageschläuche überragen endseitig dieses erste Lumen.

Im Hinblick auf eine möglichst genaue Positionierung der Drainageschläuche in den jeweiligen Lungenflügeln ist der Intubationsschlauch vor dem Einführen in die Lunge mit Führungsdrähten versehen. Dabei ist jedem Drainageschlauch ein bis zu dem freien Ende des Drainageschlauchs reichender Führungsdraht zugeordnet, der aus dem Drainageschlauch herausziehbar sein kann. Ein solcher Führungsdraht wird beispielsweise bei minimalinvasiven kardiovaskulären Eingriffen genutzt, um einen vaskulär eingeführten Schlauch in die gewünschte Position zu bringen bzw. innerhalb des Körpers auszurichten. An diesen Vorbildern orientiert sich die hier diskutierte Weiterbildung.

Der Führungsdraht kann abhängig von seinem Durchmesser in die Wand des Drainageschlauches eingebracht oder innerhalb des Drainageschlauches verlegt sein und mit dem Drainageschlauch verbunden sein. Der Drainageschlauch hat einen relativ geringen Außendurchmesser, um möglichst tief in den linken bzw. rechten Lungenflügel eingebracht zu werden. Der Drainageschlauch sollte ausreichend steif sein, um ein Kollabieren aufgrund des Unterdrucks zu verhindern. Der effektive Strömungsdurchmesser der Drainageschläuche sollte im Hinblick auf einen möglichst geringen Strömungswiderstand möglichst groß sein. Die Auswahl des Führungsdrahtes und die Anordnung in dem Drainageschlauch sollte diesen Vorgaben bestmöglich entsprechen. Der Führungsdraht kann zur Erhöhung der Knicksteifigkeit gebraided, d.h. mit einer umfänglichen dünnwandigen Versteifung versehen sein. Eine entsprechende Versteifung kann auch der Drainageschlauch aufweisen. Die Eigensteifigkeit des Drainageschlauches sollte beispielsweise durch die Materialbeschaffenheit des den Drainageschlauch bildenden Materials und/oder beispielsweise in eine Kunststoffmatrix eingebrachte Versteifungselemente so ausgebildet sein, dass der Drainageschlauch insbesondere beim Saugen nicht kollabiert und einen hinreichenden Durchlass von Fluid während des Absaugens ermöglicht.

Ein optischer Leiter im Drainageschlauch ist ferner denkbar, um die Lage des Schlauches und der Führungsdrähte zu kontrollieren. Dieser optische Leiter sollte bis zu dem distalen Ende des Drainageschlauches reichen, um auch eine Inspektion der Lunge, insbesondere der rechten und linken Hauptbronchien zu ermöglichen.

Alternativ oder zusätzlich zu den Führungsdrähten kann der zumindest eine Drainageschlauch einen gebogenen elastischen Abschnitt, insbesondere am freien Ende des Drainageschlauches, aufweisen. Der elastische Abschnitt ist vorteilhaft, da er beim Einführen durch einen Kanal oder Lumen verformbar ist, aber seine gebogene Form wieder einnimmt, wenn der Drainageschlauch den Bereich des Kanals oder Lumens verlassen hat. Üblicherweise ist lediglich das distale Ende des Drainageschlauches gebogen. Eine Biegung bedeutet hierbei, dass der gebogene elastische Abschnitt relativ zu dem üblichen geradlinigen Verlauf des Drainageschlauches abweicht, sodass der Drainageschlauch eine einseitig abgehende Krümmung an seinem Ende hat. Eine gebogene Form an dem zumindest einen Drainageschlauch kann für die Orientierung und/oder Positionierung verwendet werden.

Im Hinblick auf eine möglichst schonende Einbringung der Drainageschläuche in die Lungenflügel wird gemäß einer bevorzugten Weiterbildung der vorliegenden Erfindung vorgeschlagen, die Drainageschläuche mit einer Beschichtung, insbesondere einer antihaftenen hydrophilen oder hydrophoben Beschichtung, zu versehen, die den Reibkoeffizienten an der Innen- und/oder Außenumfangsfläche des Drainageschlauches vermindert. Eine Beschichtung in diesem Sinne ist eine permanent mit dem Drainageschlauch verbundene Beschichtung und verhindert das Verkleben des Schlauches durch proteinhaltige Sekrete. Zusätzlich kann unmittelbar vor der Benutzung der Drainageschlauch durch Aufsprühen oder Einreiben mit einem reibungsvermindernden Fluid für das Einführen in den Lungenflügel vorbereitet werden. Eine Beschichtung an der Außenumfangsfläche verbessert das Eindringen in die Luftröhre bzw. Teile der Lunge. Eine Beschichtung an der Innenumfangsfläche verbessert das Abfördern von Sekret durch den Drainageschlauch.

Zur externen Kontrolle des Einführens und der Lage des Drainageschlauches hat dieser eine detektierbare Markierung. Die Markierung ist beispielsweise nicht-invasiv im Körper angeordnet, wobei ebenfalls der Einsatz eines Bronchoskops denkbar ist. Bei dieser Markierung kann es sich beispielsweise um einen metallischen Ring handeln, der über ein Röntgengerät detektiert werden kann, wodurch sich Informationen zur Lage des Drainageschlauches in der Lunge ableiten lassen.

Gemäß einer weiteren bevorzugten Ausgestaltung der vorliegenden Erfindung hat der Drainageschlauch zumindest eine an dem Umfang des Drainageschlauches vorgesehene Drainageöffnung. In der Regel sind über die Länge des Drainageschlauchs eine Vielzahl von Drainageöffnungen vorgesehen, die jeweils auf dem Umfang des Drainageschlauchs verteilt sind. Dadurch wird sichergestellt, dass der Drainageschlauch auch bei einer Verstopfung einer einzelnen Drainageöffnung weiterhin Flüssigkeit aus der Lunge absaugen bzw. in diese einbringen kann.

Die erfindungsgemäße Vorrichtung hat in an sich bekannter Weise ein Beatmungsgerät, das mit einem ersten Lumen des Intubationsschlauches verbindbar ist. Die Vorrichtung hat eine Saugpumpe und eine optionale Spülpumpe, die an wenigstens einer Ventileinrichtung, beispielsweise an 3/3 Wegeventile oder zuminest zwei Einzelventile, angeschlossen sind. Diese Ventileinrichtungen sind dazu ausgebildet drei Mechanismen zuzulassen, nämlich um (i) Spülen zu können, (ii) Saugen zu können oder (iii) einen Durchfluss zu verhindern, wozu beide Ventile verschlossen werden können. Sie verbinden die Saug- und die optionale Spülpumpe mit einer Schnittstelle für den wenigstens einen Drainageschlauch. Die erfindungsgemäße Vorrichtung hat eine Steuerung, die steuerungsmäßig mit der wenigstens eine Ventileinrichtung, der Saugpumpe und der optionalen Spülpumpe verbunden ist. Die Steuerung steuert dabei durch stellen der Ventileinrichtungen und Ansteuern der Saugpumpe und der optionalen Spülpumpe die Abfolge von Spülen und Saugen in den Lungenflügeln. Auf eine Spülpumpe kann beispielsweise verzichtet werden, wenn die Spülflüssigkeit mittels Gravitation in die Lunge eingeleitet wird.

Die Ventileinrichtungen können die Saug- und Spülpumpe mit dem jeweiligen Drainageschlauch wahlweise verbinden, sodass entweder gespült oder durch die gleiche Leitung drainiert, d.h. abgesaugt werden kann. Die entsprechenden Ventileinrichtungen sind ferner so ausgebildet, dass sie den Drainageschlauch proximal verschließen können, sodass deren Strömungswege die Beatmung durch das Beatmungsgerät nicht beeinflussen.

Die erfindungsgemäße Vorrichtung hat bevorzugt einen in einem Leitungsweg der Spülpumpe angeschlossenen Drucksensor, dessen Signal in der Steuerung verarbeitet wird. Dieser Drucksensor lässt beispielsweise Blockaden erkennen, wenn Spülflüssigkeit zwar durch die Spülpumpe gepumpt wird, allerdings nicht bis zu dem Ende des zugeordneten Drainageschlauches gelangt. Dieses Ereignis kann dazu führen, dass die Steuerung ein optisches oder akustisches Signal absetzt, welches auf eine Störung hinweist. Ein entsprechendes Ereignis kann auch dazu führen, dass die Saug- und Spülfunktion automatisiert abgestellt wird.

Bevorzugt hat die Vorrichtung einen in einem Leitungsweg der Saugpumpe angeschlossenen Durchflussmesser, dessen Signal in der Steuerung verarbeitet wird. Der Durchflussmesser ermittelt das abgesaugte Volumen und dient der Überwachung, damit die Beatmung nicht beeinträchtigt wird. Ferner gibt dieser Aufschluss über auffällige Verstopfungen im Drainageschlauch. Dabei befindet sich der Durchflussmesser üblicherweise in Strömungsrichtung hinter einem Auffangbehälter für das abgesaugte Sekret aus der Lunge. So misst der Durchflussmesser das Volumen der aus der Lunge abgesaugten Luft ohne die flüssigen Bestandteile und kann somit Auskünfte über den Heilungsverlauf geben. Des Weiteren kann über einen solchen Durchflussmesser eine Fehlfunktion erkannt werden, wenn durch den Drainageschlauch ein zu hohes Volumen aus der Lunge abgeleitet wird, was den Verdacht begründet, dass die zur Beatmung eingebrachte Luft unmittelbar durch den Drainageschlauch abgesaugt wird.

Der besagte Auffangbehälter ist dabei wie ein üblicher Sekret-Auffangbehälter ausgebildet, wie er beispielsweise bei der Vakuum-Therapie zum Einsatz kommt. Zu jeder Drainageleitung kann ein einzelner Saugbehälter vorgesehen sein, sodass sich aus den jeweils individuell angesammelten Volumina an Sekret auch eine Aussage über den Zustand der einzelnen Lungenflügel ableiten lässt.

Weiter bevorzugt weist der Intubationsschlauch einen Drucksensor auf, insbesondere distal im Bereich des Cuffs. Mit einem solchen Drucksensor kann der Druck des Cuffs oder der Druck der durchströmenden Flüssigkeit oder des Gases im Schlauch oder der Druck in der Lunge kontrolliert werden.

Weiter betrifft die Erfindung eine Trachealkanüle zur Behandlung von Lungenkrankheiten, die zum Durchführen eines Drainageschlauches ausgebildet ist, der beispielsweise mit einer beschriebenen Vorrichtung eines Beatmungsgerätes verbindbar ist. Die Trachealkanüle weist einen Dichtungskörper im proximalen Abschnitt der Kanülenöffnung auf, durch den der Drainageschlauch hindurchführbar ist. Der Dichtungskörper ist so ausgebildet, dass dieser keine Leckluft zwischen Drainageschlauch und der Trachealkanüle hindurchströmen lässt. Zusätzlich lässt der Dichtungskörper keine Leckluft durch die Trachealkanüle strömen, wenn sich der Drainageschlauch nicht in der Trachealkanüle befindet. Vorteilhafterweise ist der Schlauch trotz Dichtungskörper axial verschiebbar und vorzugsweise zusätzlich rotierbar. Es ist ebenfalls denkbar, dass der Dichtungskörper mehrere elastische Öffnungen zum Durchführen jeweils eines Schlauches aufweist, sodass mindestens zwei Schläuche durch die Trachealkanüle durchgeführt werden können und trotzdem keine Leckluft durch die Trachealkanüle strömen kann. Denkbar ist ferner mindestens ein an der Trachealkanüle seitlich angeordnetes Fixierelement, das die Trachealkanüle im Trachealbereich fixiert und aus einem elastischen und einem nicht-elastischen Teilelement besteht. Das äußere, distal vorgesehene nicht-elastische Teilelement verhindert ein zu weites Eindrücken der Trachealkanüle in an sich bekannter Weise verhindert, wohingegen das elastische Teilelement beim Einschieben beispielsweise parallel zu dem Hauptkörper der Trachealkanüle angeordnet ist und durch die Haut hindurch geschoben werden kann. Dieses elastische Teilelement stellt sich nach dem Einführen der Trachealkanüle aufgrund von elastischen Rückstellkräften zurück. Dementsprechend ist ein Halsbereich zwischen dem elastischen Teilelement und dem nicht-elastischen Teilelement abgedichtet. Die beiden Teilelemente sind bevorzugt scheibenförmig ausgebildet. Weiter bevorzugt weist die Trachealkanüle ein Ventil auf, das es ermöglicht, einen Absaugschlauch zu entfernen, ohne dass Leckluft durch die Trachealkanüle strömen kann. Die Trachealkanüle kann alternativ verwendet werden, um eine Beatmung und Absaugung von entstandenen Sekreten im Bereich der Lungenflügel durch einen Luftröhrenschnitt zu ermöglichen.

Der Innendurchmesser der erfindungsgemäßen Trachealkanüle ist auf den zuvor erwähnten Außendurchmesser des Drainageschlauches angepasst. Konkret sollte der Innendurchmesser der erfindungsgemäßen Trachealkanüle das Hindurchführen des Drainageschlauches gerade erlauben. Der Drainageschlauch kann für sich bis in die Lunge in der zuvor beschriebenen Weise verlegt sein, d.h. ohne dass zusätzlich ein zur Beatmung eingebrachter Intubationsschlauch vorgesehen ist. Der Drainageschlauch hat dabei bevorzugt einen Cuff, der gegenüber der Trachea abdichtet, sodass ein an dem distalen Ende des Drainageschlauches wirkender Unterdruck allein in der Lunge wirkt und nicht durch Saugen in Richtung auf die Trachea ganz oder teilweise unwirksam wird.

Die zuvor erwähnten Weiterbildungen für den Intubationsschlauch gelten auch für den in dieser Weise verlegten Drainageschlauch. Namentlich kann der Drainageschlauch endseitig gekrümmt sein, um diesen nach Wahl in den linken oder rechten Bronchios einzubringen. Der Drainageschlauch kann eine Versteifung aufweisen, sodass dessen Knicksteifigkeit erhöht ist. Der Drainageschlauch kann eine detektierbare Markierung und/oder eine reibmindernde Beschichtung und/oder distal eine oder mehrere auf dem Umfang verteilte Drainageöffnungen aufweisen. Auch kann ein Drucksensor insbesondere distal des Cuffs vorgesehen sein, mit welchem der Druck in der Lunge überprüfbar ist. Diese Messgröße kann zur Steuerung der Intensität und Dauer des Absaugens über den Drainageschlauch genutzt werden.

Ein solcher Drainageschlauch eignet sich insbesondere für die Durchführung des nachfolgend beschriebenen erfindungsgemässen Verfahrens.Ferner betrifft die Erfindung ein Verfahren zur Behandlung von Lungenkrankheiten mit einem beschriebenen Intubationsschlauch, umfassend folgende Schritte: Einführen des Drainageschlauches für sich oder zusammen mit dem Intubationsschlauch, wobei der Drainageschlauch bis in den Bereich eines Lungenflügels reicht undEntfernen der sich in dem Lungenflügel gesammelten Flüssigkeit durch einen über den Drainageschlauch wirkenden Unterdruck. Zusätzlich zur Sekretabsaugung ist ein Spülen bevorzugt denkbar. Dazu sind Spülzyklen möglich, bei denen über jeweils einen der Drainageschläuche Spülflüssigkeit in einen Lungenflügel eingebracht und gegebenenfalls nach einer gewissen Einwirkzeit wieder abgesogen wird. Als Spülflüssigkeit kann beispielsweise Kochsalzlösung, eine enzymhaltige Flüssigkeit, antibiotische- oder virostatische Flüssigkeiten, entzündungshemmende Flüssigkeiten oder andere Medikamente zum Einsatz kommen, die in der Lunge befindlichen Schleim lösen, so dass dieser beim Absaugen der Spülflüssigkeit zusammen mit der Spülflüssigkeit aus der Lunge entfernt werden kann.

Weitere Einzelheiten und Vorteile der vorliegenden Erfindung ergeben sich aus der nachfolgenden Beschreibung von Ausführungsbeispielen in Verbindung mit der Zeichnung. In dieser zeigen:
- Figur 1: eine schematische Ansicht eines Intubationsschlauches;
- Figur 2: eine beispielhafte Querschnittsansicht des Intubationsschlauches nach Figur 1;
- Figur 3: das vergrößerte Detail nach Figur 1,
- Figur 4: eine schematische Darstellung der Komponenten eines Ausführungsbeispiels einer Vorrichtung zur Behandlung von Lungenkrankheiten der vorliegenden Erfindung,
- Figur 5: eine beispielhafte Querschnittsansicht einer Trachealkanüle mit einem Drainageschlauch und
- Figur 6: beispielhaft einen Dichtungskörper in Querschnittsansicht in 6a und 6b sowie eine Längsschnittansicht in 6c.

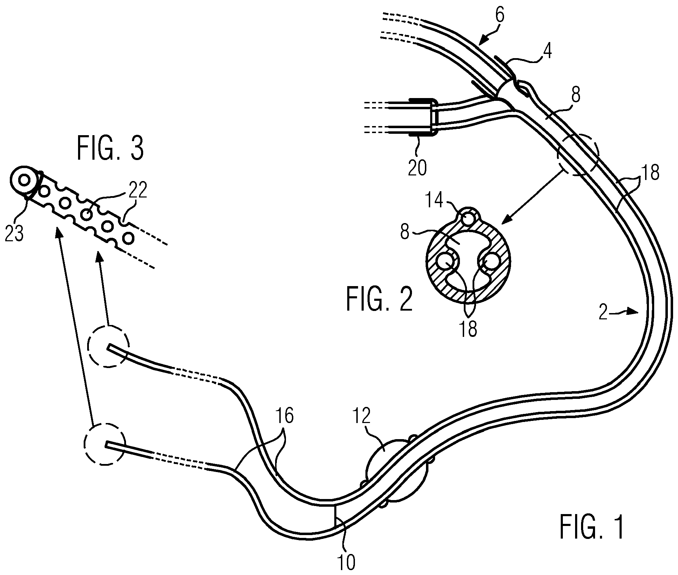

Die Figuren 1 bis 3 verdeutlichen einen Intubationsschlauch 2 mit einem Anschluss 4 zu einem Beatmungsgerät, was vorliegend durch einen Verbindungsschlauch 6 angedeutet ist. Der Intubationsschlauch 2 definiert ein erstes Lumen für die Beatmung, dass mit dem Verbindungsschlauch 6 verbunden und mit Bezugszeichen 8 gekennzeichnet ist. Das erste Lumen 8 endet bei Bezugszeichen 10 distal. Unmittelbar benachbart zu diesem distalen Ende 10 befindet sich ein Cuff 12. Diesem Cuff 12 ist ein zweites Lumen 14 zugeordnet, welches parallel zu dem ersten Lumen 8 verlegt ist und proximal mit einem nicht gezeigten Anschluss endet, an den beispielsweise eine Spritze angeschlossen werden kann, um den Cuff 12 durch Einbringen eines Fluids aufzuweiten.

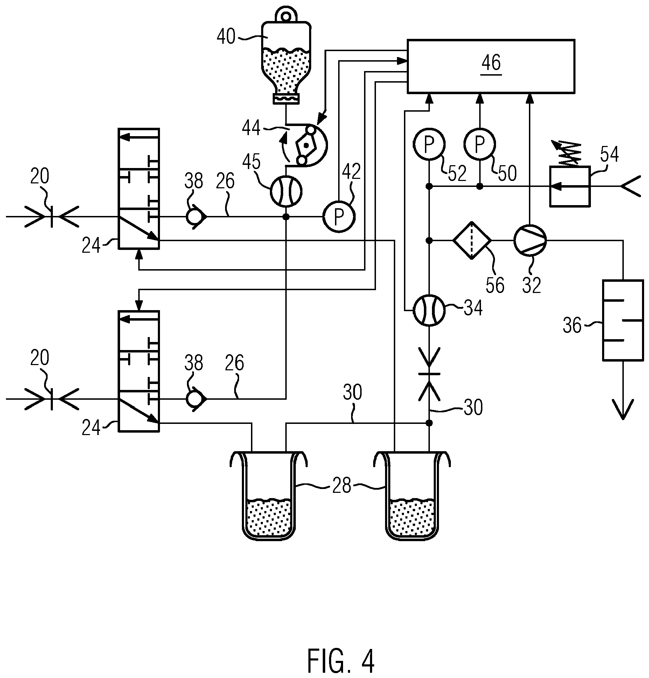

Das distale Ende 10 des ersten Lumens 8 wird von zwei Drainageschläuchen 16 überragt, die mit einem dritten Lumen 18 kommunizieren. Wie Figur 2 verdeutlicht, sind zwei solcher dritter Lumen 18 wie auch das zweite Lumen 14 in dem Intubationsschlauch 2 verwirklicht. An dem proximalen Ende sind zu diesem dritten Lumen 18 Anschlussleitungen 20 vorgesehen, die in der nachstehend unter Bezugnahme auf Figur 4 beschriebenen Weise angeschlossen sind.

Das freie Ende der Drainageschläuche 16 ist in Figur 3 dargestellt. Neben dem freien Ende des Drainageschlauchs 16, welches eine Öffnung ausbildet, sind auf dem Umfang verteilt und mit vorbestimmter axialer Länge an dem Drainageschlauch 16 eine Vielzahl von Drainageöffnungen 22 ausgebildet. In einem Endbereich jedes Drainageschlauchs 16 befindet sich eine Markierung 23 in Form eines in den Drainageschlauch 16 eingearbeiteten metallischen Rings zur Detektion der Lage des Drainageschlauchs 16 in dem Lungenflügel.

Die Figur 4 zeigt ein Ausführungsbeispiel einer Vorrichtung zur Behandlung von Lungenkrankheiten mit zwei Ventileinrichtungen 24 in Form von 3/3 Wegeventilen, an die jeweils die zuvor erwähnten Anschlussleitungen 20 angeschlossen sind. Auf der gegenüberliegenden Seite sind diese 3/3 Wegeventile mit einer Absaugleitung 30 verbunden, die jeweils zu einem Sammelbehälter 28 führt, die zu einer Saugpume 32 führt, wobei zwischen den Sammelbehältern 28 und der Saugpumpe 32 bei dem gezeigten Ausführungsbeispiel ein Durchflussmesser 34 vorgeschaltet ist, der das Volumen des abgesaugten gasförmigen Fluid misst. Ein Filter 56 ist zwischen den Sammelbehältern 28 und der Saugpumpe 32 angeordnet, der das Volumen entsprechend filtern kann. Die Saugpumpe 32 führt über einen Schalldämpfer 36 und/oder einen antibakteriellen beziehungsweise antiviralen Filter das abgesaugte Gas an die Atmosphäre ab.

Dieses Gas kommt aus der Lunge und wird üblicherweise durch das nicht gezeigte Beatmungsgerät, welches Teil der Vorrichtung ist, über das erste Lumen 8 in die Lunge eingebracht.

Die 3/3 Wegeventile sind ferner an eine Spülleitung 26 angeschlossen, die unter Zwischenschaltung eines Rückschlagventils 38 mit einem Reservoir 40 für Spülflüssigkeit kommuniziert. An einem Abzweig zu den 3/3 Wegeventile ist ein Drucksensor 42 der Spülleitung 26 zugeschaltet, über welchen der Innendruck in der Spülleitung 26 gemessen werden kann. Ein übermäßiger Druckanstieg signalisiert eine Verstopfung der Spülleitung 26 bzw. der dem 3/3 Wegeventil nachgelagerten Drainageleitung 16. Zwischen dem Drucksensor 42 und in dem Reservoir 40 befindet sich eine Schlauchpumpe 44, die ein Ausführungsbeispiel einer Spülpumpe der vorliegenden Erfindung ist. Denkbar ist ferner, dass die Spülflüssigkeit ohne den Einsatz einer Schlauchpumpe 44 über die Schwerkraft erfolgt. Stromabwärts der Schlauchpumpe 44 ist ein Durchflussmesser 45 angeordnet.

Mit Bezugszeichen 46 ist eine Steuerung gekennzeichnet, die datenmäßig mit der Schlauchpumpe 44, dem Drucksensor 42 und den beiden 3/3 Wegeventilen verbunden ist und die 3/3 Wegeventile stellt. Die Steuerung 46 ist ferner verbunden mit der Absaugpumpe 32, dem Durchflussmesser 34 und einem mit der Absaugleitung 30 verbundenen Drucksensor 50, der ebenfalls datenmäßig mit der Steuerung 46 verbunden ist. Dem Drucksensor 50 ist ein Manometer 52 zugeordnet, mit dem der Innendruck insbesondere beim manuellen Einstellen des Saugdrucks über den unmittelbar benachbart zu dem Manometer 52 in die Absaugleitung 30 eingebauten Druckregler 54, wobei der Druckregler 54 über die Steuerung 46 reguliert werden kann.

In der Steuerung 46 ist ein Programm hinterlegt, welches die 3/3 Wegeventile jeweils separat ansteuern kann, um diese für den Durchgang eines Fluid zu sperren oder wahlweise mit der Spülleitung 26 bzw. der Absaugleitung 30 zu verbinden. Die Steuerung ist üblicherweise so eingestellt, dass eines der 3/3 Wegeventile den Durchgang von Fluid sperrt, während das andere der 3/3 Wegeventile zunächst Spülflüssigkeit in den Drainageschlauch 16 einleitet und nach einer gewissen vorbestimmten Spüldauer umschaltet, sodass die in die Lunge eingebrachte Flüssigkeit zusammen mit Luft aus der Lunge abgezogen und in dem zugeordneten Sammelbehälter 28 die Flüssigkeit von dem Gas getrennt wird.

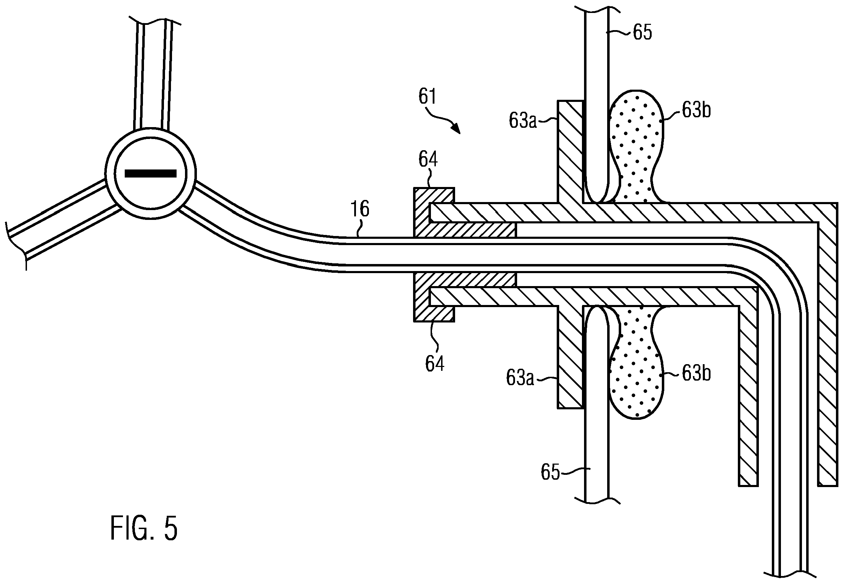

Figur 5 zeigt eine Trachealkanüle 61, in die ein Drainageschlauch 16 eingeführt und in der dieser sowohl rotiert als auch axial verschoben werden kann. Die Trachealkanüle 61 ist so ausgebildet, dass der Drainageschlauch 16 in der Trachealkanüle 61 eine Richtungsänderung erfährt, wodurch der Drainageschlauch entlang der Luftröhre bis in den Bronchienbereich eingeschoben werden kann. Im proximalen Bereich der Kanülenöffnung weist die Trachealkanüle 61 einen Dichtungskörper 64 auf, der verhindert, dass Leckluft oder Keime/Bakterien von außen eindringen können. Ferner weist die Trachealkanüle 61 zwei Fixierungselemente 63a, 63b auf, wobei das das äußere, distal vorgesehene nicht-elastische Teilelement 63a ein zu weites Eindrücken der Trachealkanüle 61 in an sich bekannter Weise verhindert, wohingegen das Teilelement 63b ein elastisches Teilelement ist, welches beim Einschieben beispielsweise parallel zu dem Hauptkörper der Trachealkanüle 61 angeordnet und durch die Haut hindurch geschoben werden kann. Dieses elastische Teilelement 63b stellt sich nach dem Einführen der Trachealkanüle 61 aufgrund von elastischen Rückstellkräften zurück. Dementsprechend ist ein Halsbereich 65 zwischen dem elastischen Teilelement 63b und dem nicht-elastischen Teilelement 63a abgedichtet. Die beiden Teilelemente 63a, 63b sind bevorzugt scheibenförmig ausgebildet.

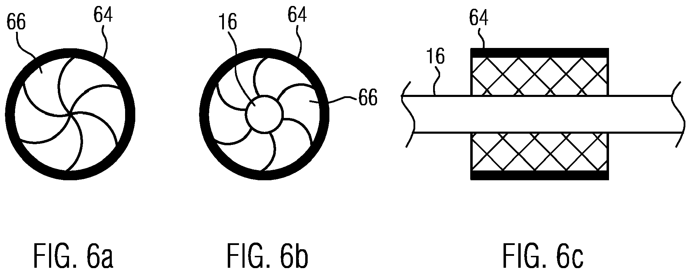

Figur 6 zeigt beispielhaft einen Dichtungskörpers 64 in Querschnittsansicht in drei Ansichten 6a, 6b und 6c. Die Ansicht 6a zeigt einen Dichtungskörper 64 ohneDrainageschlauch 16, die Ansicht 6b dieselbe Querschnittsansicht eines Dichtungskörper 64 Drainageschlauch 16 und die Ansicht 6c eine Längsschnittansicht mit einem Drainageschlauch 16.

Die Querschnittsansichten 6a, 6b zeigen das Wesen des Dichtungskörpers, der wie ein Ventil wirkt und bei Fehlen eines Drainageschlauches 16 die Trachealkanüle 61 aufgrund einer elastischen Vorspannung von Kreissegmenten 66 zwangsläufig verlegt (vgl. Figur 6a). Die Kreissegmente bestehen beispielsweise aus einem kompressiblen Kunststoff, sodass durch Kompression und Verformung der einzelnen Kreissegmente 66 von der in Figur 6a gezeigten Stellung in die in Figur 6b gezeigte Stellung im Inneren ein Freiraum gebildet werden kann, in welchem in Figur 6 Drainageschlauch 16 eingeführt ist. Die Kreissegmente 66 umgeben diesen Drainageschlauch 16 an der Außenumfangsfläche. So ist auch in diesem Fall die Trachealkanüle 61 an ihrem proximalen Ende abgedichtet, wobei lediglich der Schlauch die Trachealkanüle 61 durchdringt. Während bei der zuvor beschriebenen Ausgestaltung die Kreissegmente 66 als kompressible Kunststoffkörper in einem starren Rohr angeordnet sind, sind auch Varianten denkbar, bei welchen die die Abdichtung bewirkenden Segmente in einem expandierbaren Rohr aufgenommen sind, welches sich elastisch radial zurückstellt, wenn der Drainageschlauch 16 nicht mehr in dem Dichtungskörper aufgenommen ist.

Figur 6c verdeutlicht den in Figur 6b gezeigten Zustand. Ersichtlich hat der Dichtungskörper 64 eine erhebliche axiale Länge, sodass eine zuverlässige Abdichtung zwischen den Kreissegmenten 66 und der Außenumfangsfläche des Intubationsschlauches 2 bewirkt werden kann.

### Bezugszeichenliste

- 2: Intubationsschlauch
- 4: Anschluss
- 6: Verbindungsschlauch
- 8: erstes Lumen
- 10: distales Ende des ersten Lumen
- 12: Cuff
- 14: zweites Lumen
- 16: Drainageschläuche
- 18: drittes Lumen
- 20: Anschlussleitung
- 22: Drainageöffnung
- 23: Markierung
- 24: Ventileinrichtung
- 26: Spülleitung
- 28: Sammelbehälter
- 30: Absaugleitung
- 32: Saugpumpe
- 34: Durchflussmesser
- 36: Schalldämpfer
- 38: Rückschlagventil
- 40: Reservoir für Spülflüssigkeit
- 42: Drucksensor
- 44: Schlauchpumpe
- 45: Durchflussmesser
- 46: Steuerung
- 50: Drucksensor
- 52: Manometer
- 54: Druckregler
- 56: Filter
- 61: Trachealkanüle
- 63a: nicht elastisches Teilelement
- 63b: elastisches Teilelement
- 64: Dichtungskörper
- 65: Halsbereich
- 66: Kreissegment

## Patentansprüche

1. Intubationsschlauch (2) mit einem ersten Lumen (8) für einen Gasaustausch und einem zweiten Lumen (14) für die Steuerungen eines Cuffs (12), der von einem distalen Ende (10) des ersten Lumens (8) überragt ist, **gekennzeichnet durch** zumindest einen Drainageschlauch (16), der das distale Ende (10) des ersten Lumens (8) überragt und mit einem parallel zu dem ersten Lumen (8) verlegten dritten Lumen (16) kommuniziert.

2. Intubationsschlauch (2) nach Anspruch 1, **dadurch gekennzeichnet, dass** der zumindest eine Drainageschlauch (16) einen Außendurchmesser von 2 mm bis 10 mm, insbesondere von 2 mm bis 5 mm, hat.

3. Intubationsschlauch (2) nach Anspruch 1 oder 2 **dadurch gekennzeichnet, dass** das erste Lumen (8) mit einer derartigen Länge ausgebildet ist, dass das erste Lumen (8) im Bereich des unteren Endes der Trachea endet.

4. Intubationsschlauch (2) nach einem der vorherigen Ansprüche, **dadurch gekennzeichnet, dass** der zumindest eine Drainageschlauch (16) in Verlaufsrichtung des dritten Lumens (18) axial verschiebbar und vorzugsweise zusätzlich rotierbar ist.

5. Intubationsschlauch (2) nach einem der vorherigen Ansprüche, **dadurch gekennzeichnet, dass** der zumindest eine Drainageschlauch (16) ein bis zu dem freien Ende des Drainageschlauches (16) reichender Führungsdraht zugeordnet ist.

6. Intubationsschlauch (2) nach einem der vorherigen Ansprüche, **dadurch gekennzeichnet, dass** der zumindest eine Drainageschlauch (16) einen gebogenen elastischen Abschnitt, insbesondere am freien Ende des Drainageschlauches (16), aufweist.

7. Intubationsschlauch (2) nach einem der vorherigen Ansprüche, **dadurch gekennzeichnet, dass** der wenigstens eine Drainageschlauch (16) mit zumindest einer an dem Umfang des Drainageschlauchs (16) ausgebildeten Drainageöffnung (22) versehen ist.

8. Intubationsschlauch (2) nach einem der vorherigen Ansprüche, **dadurch gekennzeichnet, dass** der wenigstens eine Drainageschlauch (16) mit einer detektierbaren Markierung (23) versehen ist.

9. Intubationsschlauch (2) nach einem der vorherigen Ansprüche, **dadurch gekennzeichnet, dass** der Drainageschlauch (16) mit einer Reibkoeffizient-vermindernden Beschichtung, insbesondere einer antihaftenen hydrophilen oder hydrophoben Beschichtung, versehen ist.

10. Intubationsschlauch (2) nach einem der vorherigen Ansprüche, **dadurch gekennzeichnet, dass** der Intubationsschlauch (2) einen Drucksensor aufweist, insbesondere distal im Bereich des Cuffs.

11. Vorrichtung zur Behandlung von Lungenkrankheiten mit einem Beatmungsgerät, das mit einem ersten Lumen (8) eines Intubationsschlauches (2) verbindbar ist, einer Saugpumpe (32), die an zumindest einer Ventileinrichtung (24) angeschlossen ist, die die Saugpumpe (32) mit einer Schnittstelle (20) für Drainageschläuche (16) verbindet, und einer Steuerung (46), die steuerungsmäßig mit der mindestens einen Ventileinrichtung (24) und der Saugpumpe (32) verbunden ist.

12. Vorrichtung nach Anspruch 11 **gekennzeichnet durch** eine Spülpumpe (44), die an der mindesten einen Ventileinrichtung (24) angeschlossen ist, die die Spülpumpe (44) mit einer Schnittstelle (20) für Drainageschläuche (16) verbindet und die Spülpumpe (44) mit der Steuerung (46) verbunden ist und einen in einem Leitungsweg (38) der Spülpumpe (44) angeschlossenen Drucksensor (42), dessen Signal in der Steuerung (46) verarbeitet wird.

13. Vorrichtung nach Anspruch 11 oder 12 **gekennzeichnet durch** einen in einem Leitungsweg (30) der Saugpumpe (32) angeschlossenen Durchflussmesser (34), dessen Signal in der Steuerung (46) verarbeitet wird.

14. Vorrichtung nach einem der Ansprüche 11 bis 13 **gekennzeichnet durch** einen Intubationsschlauch nach einem der Ansprüche 1 bis 10, dessen erstes Lumen an das Beatmungsgerät und dessen Drainageschläuche (16) an die Ventileinrichtung (24) angeschlossen sind.

15. Trachealkanüle (61) zur Behandlung von Lungenkrankheiten, ausgebildet zum Durchführen eines Drainageschlauches (16), der mit einer Vorrichtung nach einem der Ansprüche 11 bis 14 verbindbar ist, **gekennzeichnet durch** einen Dichtungskörper (64) im proximalen Abschnitt der Kanülenöffnung, durch den der Drainageschlauch (16) hindurchführbar ist.
